# EUROPEAN PATENT APPLICATION

(11) **EP 1 239 232 A1**
(43) Date of publication of application: **11.09.2002**
(21) Application number: 02251553.0
(22) Date of filing: 06.03.2002
(51) Int. Cl.: F24F 3/14, E03B 3/28, A61L 2/10

(54) **Air treatment unit**

(30) Priority: 07.03.2001 GB 0105636
(71) Applicant: Garvin, William Joseph, London SW14 8SP (GB)
(72) Inventor: Garvin, William Joseph, London SW14 8SP (GB)
(74) Representative: Butler, Lance

(57) **Abstract**

An air treatment unit is characterised by an inlet filter comprising both an electrostatic filter (16) and a finely porous filter (17) to remove all airborne solids having a particle size of more than 0.5 µm and in that both the condensation chamber (36) and the reservoir (70) contain ultraviolet sources (40, 72).

## Description

This invention relates to a system for treating air to extract moisture and entrained solid particulate material. Additionally it provides for treatment, collection and storage of the extracted moisture as potable water.

Air purification filters are well-established products and there have also been many proposals for dehumidifiers to extract moisture from the air. In some instances the moisture removal is an end in itself, so as to reduce atmospheric moisture to more comfortable levels, but it has also been proposed to recover and collect the extracted moisture to provide a source of potable water.

US patent No. 5,106,512 describes a water generating device for obtaining potable water from ambient air. The device includes air ducting, an filter, a condenser for extracting water vapour from the air, a fan to draw air through the condenser, an internal container to receive collected water, a water pump, a water filter, an intermediate holding reservoir between the condenser and collection point, and ultraviolet light means for killing microorganisms in the extracted and filtered water.

US patents Nos. 5,669,221 and 5,845,504, the latter being a continuation in part of the former, describe a potable water recovery and dispensing system including air inlet and exhaust ports, filtration systems to remove particulates and aerosols from the incoming air, a heat absorber to cool the filtered air to its dew point to form liquid water, a reservoir for the collected water, and a bacteriostatic loop comprising an activated carbon filter and an ultra-violet treatment zone, through which loop water is pumped at a predetermined flow rate to kill adventitious bacteria and viruses.

As is recognised in the prior art, thorough filtration of the air and thorough bactericidal treatment of the collected water are necessary features of systems put on the domestic and industrial markets. It is an objective of the present invention to achieve further significant improvements in respect of both these features

According to the present invention there is provided an air treatment unit which comprises an air inlet filter, a condensation chamber, a chilling surface within the condensation chamber, a fan to draw atmospheric air into the unit, a refrigerant circuit to convey cold refrigerant to the chilling surface, moisture collection means, a reservoir for collected water, and an ultra-violet source for destruction of bacteria, characterised in that the inlet filter comprises both an electrostatic filter and a finely porous filter to remove all airborne solids having a particle size of more than 0.5 µm and in that both the condensation chamber and the reservoir contain ultra-violet sources.

The units according to the invention serve a triple purpose. They clean dust and other solid particles from the air to a very high level of purity, providing a healthy ambient atmosphere, especially for people suffering from hay fever or other allergies. They dehumidify the air, making for more comfortable ambient conditions, especially in tropical climates, and they collect and store a ready supply of clean bacteria-free drinking water.

The units can be manufactured in different sizes according to the required duty. Domestic units for the home or small offices suitably have a water storage capacity of 18.9 litres (5 US gallons). Larger units for large homes, offices or restaurants can suitably have up to 75 litres (about 20 US gallons) storage capacity. A particularly useful version of unit according to the invention is a self-contained emergency unit for use in disaster relief and having its own power source, fuelled by gas, diesel, LPG or solar energy, with a storage capacity of up 190 litres (about 50 US gallons).

To ensure the highest quality of air dispensed from the unit the inlet filter preferably is of a high specification to remove all airborne solids having a particle size down to 0.5 µm, preferably 0.3 µm. Such high filtration levels are preferably achieved by the use of both an electrostatic filter and a finely porous ("HEPA") filter.

Filters are also desirably used for cleaning the collected water and these too should be of a high specification.

The presence of ultra-violet sources, typically lamps, in at least the condensation chamber and the reservoir assist in ensuring the destruction of airborne and waterborne bacteria.

The presence of an ultra-violet source in the condensation chamber is thus am important aspect of the invention. Any micro-bacteria are killed as the moisture collects on the chilling surface in the condensation chamber. The water from the condensation chamber is then preferably passed through a two-stage carbon/bacteria filter to remove any remaining odours or other bacteria.

Water sent to the reservoir is preferably in constant exposure to another ultra-violet source, thereby preventing any bacterial growth.

Chilling of the incoming air is effected by a conventional refrigeration cycle of expansion and compression of refrigerant (typically fluocarbons R22 and R134) in a closed system. The inherent moisture (relative humidity) in the air forms a "cloud" in the condensation chamber, condenses on the chilling surface and is collected and stored. However treatment of the water is essential to ensure that it is safe for human consumption. It is preferably filtered prior to storage

The chilling surface can be any form of refrigeration heat exchanger: evaporator coils, or flat or corrugated plate exchangers. They are best made of metal, typically aluminium, copper, nickel or stainless steel, or alloys thereof. The portions of the heat exchanger that are contacted by moisture to be collected are preferably coated with a nonporous, non-corrosive material, which may be a corrosion-resistant metal (for example nickel-plated copper cooling coils) or a tough inert plastic such as polytetrafluorethylene (PTFE/"Teflon"™). The coating helps to resist growth of bacteria and adds to the efficiency of the system.

In a preferred embodiment of the invention, a heat exchanger which may be similar to the heat exchanger used for the chilling surface, is included within the unit in a second chamber similar to the condensation chamber but at the downstream side of the fan. The heat exchangers in the condensation and second chambers are thus located opposite each other with the fan, located in baffle plates, between them. On the one side is the chilling heat exchanger and on the other side is a condenser. Optionally a further ultra-violet lamp can be located in the second chamber.

The fan is preferably bi-directional and is preferably of variable speed. These features are beneficial in making the unit suitable for use in a wide range of local climates.

The unit preferably includes sensors to detect the operating and storage conditions, thereby enabling checks to ensure that it is operating as intended. It preferably also includes a control panel to monitor and control all on-off actions and sensed parameters.

Preferably the sensors include temperature gauges associated with the chilled and warmed heat exchangers, thereby enabling balance of the heat exchangers to their most efficient state, for example in some circumstances by returning warmed air from the second chamber to the condensation chamber.

The water storage reservoir preferably includes a water level indicator, most preferably a float-switch to shut down the unit when the reservoir is full.

The unit preferably includes an alarm to indicate if the mains power source is disabled.

The invention is further described, in a non-limiting manner, with reference to the accompanying figures, in which:

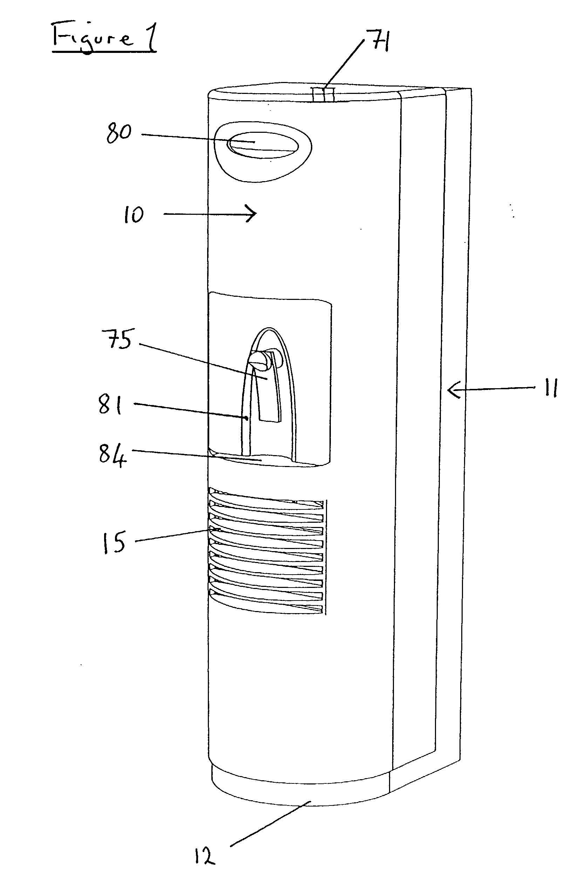

**Figure 1** is a perspective view of the exterior of a domestic air treatment unit according to the invention, and

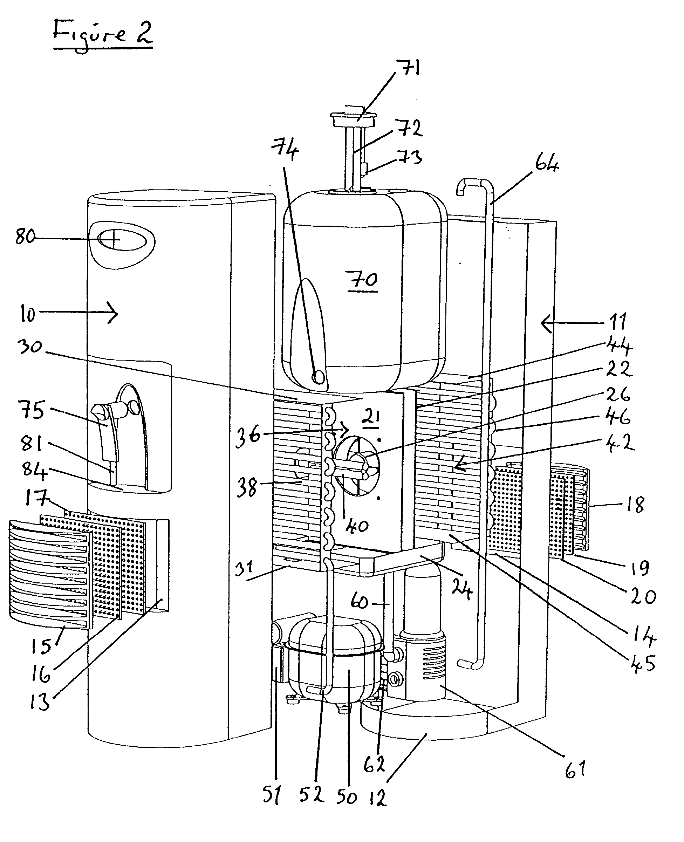

**Figure 2** is a perspective view of the unit of Figure 1 but shown as an exploded view so as to reveal the internal components.

The unit comprises a housing formed in two parts, 10 and 11. Part 10, which is substantially U-shaped in cross section, provides the front of the housing and part 11 forms the rear. The rear housing 11 incorporates a base 12 of which the forward end has a U-shaped cross section corresponding to the U-shape of housing 10, which in the assembled form rests upon it.

The front housing 10 has an opening 13 which receives an air grill 15 and a multiple filter comprising an electrostatic filter 16 and a finely porous filter 17. The filter 16 is formed of electrostatically charged cloth.

Filter 17 is a "HEPA" filter of finely divided cloth. In combination the filters are capable of removing airborne particles as small as 0.3 µm.

The rear housing 11 similarly has an opening 14, which receives an air grill 18 and a multiple filter comprising an electrostatic filter 19 and a finely porous filter 20.

Behind the opening 13 two vertical plates 21, 22 rest on a tray 24 and support a variable speed bi-directional fan 26. Horizontal plates 30, 31 form with the vertical plate 20 and the rear of the housing 10 an enclosed chamber 36 to receive air drawn in by the fan 26. Within the chamber 36 a tubular evaporator coil 38 is located immediately behind the opening 13. The coils of the evaporator coil are formed of aluminium-finned copper tubing with a coating of polytetrafluorethylene (Teflon™). An ultra-violet lamp 40 is located within the chamber 36 between the evaporator coil 38 and the fan 26.

A second enclosed chamber 42 is formed behind the fan 26 by the vertical plate 22, the sides of the front housing 10, the rear housing 11 and horizontal plates 44, 45 equivalent to plates 30, 31. Chamber 42 contains a tubular condenser coil 46 located adjacent to the opening 14. It also includes a further ultra-violet lamp (not shown).

A refrigerant cycle within the unit comprises the condenser coil 46, an expansion valve and associated tubing (not shown), the evaporator coil 38, and a compressor 50 powered by an electric motor 51. A return tube 52 leads from the evaporator coil 38 to the compressor 50. Compressed hot refrigerant is conveyed to the condenser coil 46 by a feed tube (not shown). Cooled liquid refrigerant from the condenser coil 46 is conveyed by a connection tube (not shown) to the expansion valve, and passes as cooled vapour into the evaporator coil 38, from where it returns via tube 52 to the compressor 50.

The tray 24 has a water drainage tube 60 which leads to a water pump 61 driven by an electric motor 62. The pump 60, which has an integral two-stage carbon filter, is connected by a feed tube 64 to a translucent container 70 which rests on the plates 32 and 44 and serves as a reservoir for collected water. A cap 71 supports a water-proofed ultra-violet lamp 72 to be disposed in the interior of the container 70. The base of the container 70 has an orifice 74 to receive a lever-operated tap 75.

Sensors (not shown) are provided for the fan 26 and for all three ultraviolet lamps (40, 72 and the lamp in chamber42) to indicate any failure of these items. Temperature sensors are provided in chambers 36 and 42. The container 70 also has a float switch 73 to switch off the fan 24, the lamp 40, the lamp in chamber 42, the compressor 50 and the pump 61 when the container 70 is full of water

The front of the housing 10 has a glow panel 80 through which the container 70 and the illuminated lamp 72 can be observed. It further includes a recess 81 with an orifice 82, corresponding to the container orifice 74, through which the tap 75 can be inserted. The base 84 of the recess 81 is flat and sufficiently wide to receive a drinking vessel beneath the outlet of the tap 75.

A control box (not shown) is located in the rear panel 11. This includes switches and warning lights to indicate the status of the sensed items. The lamp 72 are kept on for the entire operational period of the unit, including periods when the fan 24 and pump 61 are not operating, and has a back-up battery supply (not shown) to guard against mains failure.

Air drawn into the chamber 36 by the fan 26 passes through the filters 16, 17 which remove solid particulate matter, odours and some bacteria. The so-filtered air contacts the chilling surface of the evaporator coil 38. The lamp 40 in the chamber 36 kills any remaining bacteria. The chamber 38 serves as a water-collection zone in which water vapour from the air condenses on the evaporator coil 38 and falls into the tray 24. From the tray 24 the water runs through tube 60 to the pump 61 to be raised to and stored in the container 70. Water reaching the container 70 should be bacteria-free but the lamp 72 ensures that any residual bacteria or any bacteria entering the container from the atmosphere are also destroyed. Dehumidified air passes through the chamber 42 and out of the unit through the filters 19, 20 and grill 18, emerging in a finely-filtered condition.

At pre-set intervals the fan 24 may be operated in the reverse direction for a short period to effect a measure of regeneration of the filters by relieving any blockages caused by dust particles or water deposited on the filters 16 and 17. At longer defined intervals all the filters are removed, cleaned and replaced. It may also be necessary to operate the fan 24 in reverse mode for short periods in cold ambient conditions in which the moisture in chamber 36 would tend to freeze. A reverse air flow conveys warm air from chamber 42 into chamber 36 to raise the temperature therein.

## Claims

1. An air treatment unit which comprises an air inlet filter, a condensation chamber, a chilling surface within the condensation chamber, a fan to draw atmospheric air into the unit, a refrigerant circuit to convey cold refrigerant to the chilling surface, moisture collection means, a reservoir for collected water, and an ultra-violet source, **characterised in that** the inlet filter comprises both an electrostatic filter (16) and a finely porous filter (17) to remove all airborne solids having a particle size of more than 0.5 µm and **in that** both the condensation chamber (36) and the reservoir (70) contain ultra-violet sources (40, 72).

2. An air treatment unit as claimed in claim 1, in which the inlet filter preferably is of a specification to remove all airborne solids having a particle size of more than 0.3 µm.

3. An air treatment unit as claimed in claim 1 or claim 2, in which the inlet filter comprises both an electrostatic filter (19) and a finely porous filter (20).

4. An air treatment unit as claimed in any preceding claim, comprising a two-stage carbon/bacteria filter to remove any remaining odours or other bacteria from water leaving the condensation chamber.

5. An air treatment unit as claimed in any preceding claim, in which the chilling surface is in the form of cooling coils.

6. An air treatment unit as claimed in any preceding claim, in portions of the chilling surface that are to be contacted by moisture to be collected are coated with a nonporous, non-corrosive material.

7. An air treatment unit as claimed in claim 6, in which the coating is selected from a corrosion-resistant metal and a tough inert plastic.

8. An air treatment unit as claimed in any preceding claim, which comprises second chamber similar to the condensation chamber but at the downstream side of the fan and which includes a heat exchanger similar to the chilling surface.

9. An air treatment unit as claimed in claim 8, in which an ultra-violet lamp is located in the second chamber.

10. An air treatment unit as claimed in any preceding claim, in which the fan is bi-directional.

11. An air treatment unit as claimed in any preceding claim, in which the fan is of variable speed.

12. An air treatment unit as claimed in any preceding claim, which includes sensors to detect the operating and storage conditions.

13. An air treatment unit as claimed in any preceding claim, which includes a control panel to monitor and control all on-off actions and any sensed parameters.

14. An air treatment unit as claimed in any preceding claim, which includes temperature gauges associated with the chilled and warmed heat exchangers, to enabling balance of the heat exchangers to their most efficient state.

15. An air treatment unit as claimed in any preceding claim, which includes a water level indicator in the water storage reservoir.

16. An air treatment unit as claimed in claim 15, in which the water level indicator is a float-switch.
